# EUROPEAN PATENT SPECIFICATION

(11) **EP 0 707 063 B1**
(45) Date of publication and mention of the grant of the patent: **17.06.1998**
(21) Application number: 95111412.3
(22) Date of filing: 20.07.1995
(51) Int. Cl.: C12N 9/80, C12P 13/00, C12P 19/26

(54) **Glycolipid ceramide deacylase**
Glycolipid-Ceramiddeacylase
Céramide déacylase de glycolipides

(30) Priority: 21.07.1994 JP 190133/94
(43) Date of publication of application: 17.04.1996
(73) Proprietor: TAKARA SHUZO CO. LTD., Fushimi-ku, Kyoto-shi, Kyoto-fu (JP)
(72) Inventor: Ito, Makoto, Fukuoka-shi, Fukuoka (JP); Kurita, Toyohisa, c/o Matsushima Haitsu, Fukuoka-shi, Fukuoka (JP); Kita, Katsuhiro, Nagasaki-shi, Nagasaki (JP)
(74) Representative: Weisert, Annekäte, Dipl.-Ing. Dr.-Ing.

(56) References cited:
- EP-A- 0 373 038
- US-A- 5 143 841
- JOURNAL OF BIOCHEMISTRY, vol. 103, no. 1, January 1988 TOKYO JP, pages 1-4, YOSHIO HIRABAYASHI ET AL. 'A novel glycosphingolipid hydrolyzing enzyme, glycosphingolipid ceramide deacylase, which cleaves the linkage between the fatty acid sphingosine base in glycosphingolipids'
- DATABASE WPI Section Ch, Week 9416 Derwent Publications Ltd., London, GB; Class B04, AN 94-131283
- DATABASE WPI Section Ch, Week 9525 Derwent Publications Ltd., London, GB; Class C95, AN 95-190185 'Prodn. of lyso-glyco-lipid for use as analytical agent ' & JP-A-07 107 988 (HIGASHIMARU SHOYU KK) , 25 April 1995

## Description

This invention relates to a novel glycolipid ceramide deacylase having a wide substrate specificity. The invention also relates to a method of enzymatic production of a lysosphingolipid using the above glycolipid ceramide deacylase.

### BACKGROUND OF THE INVENTION

There has been known an enzyme produced by a microorganism belonging to the genus *Nocardia* (JP-A-64-60379 corresponding to U.S. Patents 4,997,760 and 5,143,841; the term "JP-A" as used herein means an "unexamined published Japanese patent application") as an enzyme which acts on a ceramide in the molecule of a glycolipid and hydrolyzes the ceramide moiety into a sphingosine base and a fatty acid, thus forming a lysoglycolipid and a fatty acid.

This enzyme, which was named ceramide deacylase [Journal of Biochemistry, 103, 1 - 4 (1988)], acts on gangliosides such as GDla, GM1, GM2 and GM3, i.e., so-called acidic glycolipids but not on Gal Cer or Glc Cer. It scarcely acts on neutral glycolipids such as Lac Cer, Gb3 Cer or asialo-GM1. An enzyme capable of hydrolyzing a bond between a sphingosine base and a fatty acid of a ceramide, which is called ceramidase (EC 3.5.1.23) [Journal of Biological Chemistry, 241, 3731 - 3737 (1966); Biochemistry, 8, 1692 - 1698 (1969); Biochemica et Biophysica Acta, 176, 339 - 347 (1969); and Science, 178, 1100 - 1102 (1972)], cannot hydrolyze a bond between a sphingosine base and a fatty acid in the ceramide moiety of a glycolipid.

Namely, none of known enzymes can hydrolyze a bond between a sphingosine base and a fatty acid of the ceramide moiety of a neutral glycolipid.

In general, naturally occurring glycolipids such as sphingoglycolipids show wide molecular variety depending on a fatty acid moiety even if they have the same carbohydrate chain. For example, Forssman glycolipid (Gb5Cer) derived from horse kidney includes at least ten different molecular species depending on its fatty acid moiety. Recently, it has revealed that the existing form or antigenicity of glycolipids in a lipid bilayer are greatly influenced by their fatty acid molecular species. Thus, attention has been paid to the structure of a fatty acid in view of physiological function of a glycolipid. It has been further found that substrate specificity of enzymes relating to degradation and synthesis of sphingolipids (including glycolipids and sphingomyelin) depends on fatty acid molecular species.

In order to elucidate the above subject, it has been required to develop technique for simply and easily converting naturally occurring sphingolipids including heterologous fatty acid molecular species into glycolipids having a single fatty acid species. It has also been desired to prepare a fluorescence-labeled sphingolipid by substituting a fatty acid of a sphingolipid with a fluorescent substance since such a fluorescence-labeled sphingolipid is expected to be not only a reagent important for contributing to elucidation of intracellular metabolism or transport route of sphingolipids but also a highly sensitive substrate for enzymes synthesizing or degrading a sphingolipid.

Known methods for producing lysosphingolipids include hydrazinolysis and alkaline hydrolysis in an alcohol solvent. When a sphingolipid containing amino sugar is treated by these methods, the amide bond in the carbohydrate chain is decomposed to give di-N-acetyllysoglycolipid. In the case of a glycolipid containing a sialic acid (ganglioside), the sialic acid moiety is deacylated. Therefore, it is necessary to protect a lipid moiety of a deacylated product with a protecting group and carry out reacylation followed by deprotection. A series of these chemical operations require great labor and technical skill. In addition, it is very difficult to prepare a lyso-form of polysialoganglioside having plural sialic acids, such as GQ1b, in accordance with the conventional chemical methods. When sphingomyelin is treated by the known methods, a choline phosphate group might be possibly liberated, which reduces the yield of the final product.

A biological method for producing a lysosphingolipid is also known (JP-A-78782). According to this method, many by-products other than a desired lysosphingolipid are formed and such by-products should be removed. Thus, this method is industrially disadvantageous in that an additional removing step is required and the yield is not satisfactory.

### SUMMARY OF THE INVENTION

An object of the present invention is to provide an enzyme having a wider substrate specificity than those of the conventionally known glycolipid ceramide deacylases, in particular, a glycolipid ceramide deacylase capable of acting well on neutral glycolipids. Another object of the present invention is to provide a method of enzymatic production of a lysosphingolipid, which is useful in the field of glycolipid engineering, using the above glycolipid ceramide deacylase in an industrial scale.

The present invention relates to a glycolipid ceramide deacylase having the following physicochemical properties of:
(1) acting on a ceramide in the molecule of sphingoglycolipid and hydrolyzing it into a sphingosine base and a fatty acid, thus forming a lysoglycolipid and a fatty acid;
(2) acting on neutral glycolipids and acidic glycolipids;
(3) having an optimum pH value range of from 5 to 7;
(4) having an optimum temperature of about 40 °C;
(5) being stable within a pH value range of from 4 to 9 when treated at 5 °C for 16 hours; and
(6) being stable at 60 °C for 30 minutes.

The present invention also relates to a method of producing a lysosphingolipid which comprises treating a sphingolipid with the above glycolipid ceramide deacylase and recovering a lysosphingolipid from the reaction mixture.

To obtain enzymes relating to glycolipids, the present inventors collected various samples (soil, seawater, fresh water, etc.) and subjected these samples to screening. In the course of the screening, they have surprisingly found out a novel glycolipid ceramide deacylase activity unknown hitherto by which neutral glycolipids and acidic glycolipids can be hydrolyzed into a sphingosine base and a fatty acid, thus forming a lysoglycolipid and a fatty acid. As a result of extensive studies, the present inventors have specified a microorganism producing the enzyme of the present invention, further purified the enzyme and clarified the physicochemical properties of the same. Further, the present inventors have succeeded in efficiently producing lysosphingolipid by treating sphingolipid with the above enzyme. Thus, the present invention has been completed.

### BRIEF DESCRIPTION OF THE DRAWINGS

Fig. 1 is a diagram which shows the optimum pH value of the glycolipid ceramide deacylase obtained by the present invention. In Fig. 1, 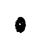 stands for the acetate buffer solution, △ stands for the phosphate buffer solution and 0 stands for the glycine buffer solution.

Fig. 2 is a diagram which shows the optimum temperature of the glycolipid ceramide deacylase obtained by the present invention.

Fig. 3 is a diagram which shows the pH stability of the glycolipid ceramide deacylase obtained by the present invention. In Fig. 3, 0 stands for the acetate buffer solution, 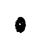 stands for the phosphate buffer solution, △ stands for the tris-hydrochloride buffer solution and □ stands for the glycine buffer solution.

Fig. 4 is a diagram which shows the heat stability of the glycolipid ceramide deacylase obtained by the present invention.

Fig. 5 is an FAB-MS spectrum of the lysoasialo-GM1 obtained by digesting asialo-GM1 with the use of the glycolipid ceramide deacylase obtained by the present invention.

Fig. 6 is an FAB-MS spectrum of the lisosphingomyelin obtained by digesting sphingomyelin with the use of the glycolipid ceramide deacylase obtained by the present invention.

### DETAILED DESCRIPTION OF THE INVENTION

The present invention will be described in greater detail below.

The method for producing the glycolipid ceramide deacylase of the present invention is not particularly restricted. Namely, it may be performed by using, for example, microorganisms or cells capable of producing the glycolipid ceramide deacylase of the present invention. For example, *Pseudomonas* sp. TK-4 can be used therefor. This strain, which has been isolated from the soil for the first time by the present inventors, has the following mycological properties.
(1) Growth temperature range: to 41 °C.
(2) Gram-staining: negative.
(3) Morphology: bacillus.
(4) Motility: positive.
(5) Growth under aerobic condition: positive.
(6) Growth under anaerobic conditions: negative.
(7) Catalase: positive.
(8) Oxidase: positive.
(9) O-F test: F.
(10) O/129 sensitivity test: nonsensitive.
(11) Purification of chromogen: +/-.
(12) Growth in ammonium ion and glucose synthetic medium: positive.
(13) Utilization of amino acid as carbon source: Arg, Asn, His, Glu, Ser and Ala.
(14) Growth in 7.5 % NaCl-containing nutrient broth: negative.
(15) Butanediol dehydrogenase activity: positive.
(16) Gas production from glycerol: positive.
(17) Gas production from glucose: positive.
(18) Evolution of hydrogen sulfide from 2.5 % aqueous solution of peptone: positive.
(19) Sugar metabolism: galactose, sucrose, arabinose.
(20) GC content: about 69.4 %.
(21) Monotrichic: yes.

Based on these results, this strain has been identified as one belonging to the genus *Pseudomonas.*

It has been named *Pseudomonas* sp. TK-4 and deposited in accordance with Budapest Treaty at National Institute of Bioscience and Human-Technology, Agency of Industrial Science and Technology, Ministry of International Trade and Industry [1-3 Higashi 1-chome, Tsukuba-shi, Ibaragi 305, JAPAN] under the accession number FERM BP-5096 since June 24, 1994.

The enzyme of the present invention can be obtained by, for example, incubating the above-mentioned strain in a nutrient medium and, after the completion of the incubation, isolating the enzyme from the culture medium. Any nutritional sources may be added to the medium, so long as the strain can utilize the same to thereby produce the enzyme of the present invention. For example, glycerol, glucose, sucrose, molasses, etc. are usable as a carbon source, while yeast extract, peptone, corn steep liquor, meat extract, defatted soybean, ammonium sulfate, ammonium nitrate, etc. are usable as a nitrogen source. Further, inorganic matters and metal salts (for example, sodium salt, potassium salt, phosphate, magnesium salt, zinc salt) may be added thereto. It is also possible to add from 0.01 to 0.5 % of a glycolipid such as asialo-GM1 to the medium so as to elevate the productivity of the enzyme of the present invention. It is also preferable to add dimethylcyclodextrin to the medium to a concentration of 0.1 %. When the strain capable of producing the enzyme of the present invention is incubated, the yield of the enzyme widely varies depending on the incubation conditions. It is generally preferable that the inoculum size of the strain ranges from 2 to 5 %, the incubation temperature ranges from 20 to 35 °C and the pH value of the medium ranges from 5 to 8. The enzyme of the present invention can be produced by incubating the strain under aeration for 1 to 7 days. Needless to say, the incubation conditions are to be set in such a manner as to achieve the maximum productivity of the enzyme of the present invention depending on the selected strain, the composition of the medium, etc.

The enzyme of the present invention is produced extracellularly by the above-described strain. Therefore, the enzyme can be purified by subjecting the culture medium to solid/liquid separation and the resulting supernatant to a purification procedure commonly employed in the art. For example, purification may be performed by salting out, precipitation from organic solvents, ion exchange column chromatography, hydroxyapatite column chromatography, gel filtration column chromatography or freeze-drying. The purity of the enzyme can be determined by, for example, polyacrylamide gel disk electrophoresis.

The glycolipid ceramide deacylase obtained by the present invention has the following enzymological and physicochemical properties.

### (1) Assay of enzyme activity

The enzyme activity of the glycolipid ceramide deacylase is assayed in the following manner. To 10 µl of a substrate solution [50 mM acetate buffer solution (pH 6.0) containing 2 mM of asialo-GM1 and 0.6 % (w/v) of Triton X100®] is added 10 µl of an enzyme solution. After reacting at 37 °C for 30 minutes, the enzyme reaction is ceased by heating the mixture to 100 °C for 3 minutes. Then the reaction mixture is concentrated to dryness with a centrifugal concentrator. Then the obtained concentrate is dissolved in 10 µl of 50 % methanol and placed on a TLC plate (silica gel 60; manufactured by Merck & Co., Inc.). After developing with chloroform/methanol/0.02 % aqueous solution of CaCl₂ (5:4:1 by volume), the glycolipid is subjected to color development by the orcinol-sulfuric acid method. With the use of this developing solvent, the glycolipid, from which the fatty acid has been eliminated, shows an Rf value somewhat later than that of the native glycolipid. This spot is determined by using a TLC chromatoscanner (Shimadzu CS-9000, manufactured by Shimadzu Corporation) at a wavelength of 540 nm.

One unit (1 U) of activity is defined as the amount of the enzyme liberating 1 µmol of lysoasialo-GM1 per minute from asialo-GM1 at 37 °C.

### (2) Function

It acts on a ceramide in the molecule of a glycolipid and hydrolyzes the ceramide moiety into a sphingosine base and a fatty acid, thus forming a lysoglycolipid and a fatty acid.

### (3) Substrate specificity

In accordance with the method of (1) for assaying the enzyme activity, the substrate specificity of this enzyme is examined. As a result, it is found out that this enzyme acts not only on acidic glycolipids of the ganglioside-series but also sphingoglycolipids of ganglioside-, globo- and lacto-series and cerebrosides composed of a monosaccharide and a ceramide binding thereto and thus forms a lysoglycolipid and a fatty acid, as shown in Table 1 below. In Table 1, GM1 and asialo-GM1 are each prepared from bovine brain [Methods in Enzymology, 83, 139 - 191 (1982)], while other substrates are marketed products manufactured by Iatron.

**Table 1**

| Substrate | Digestion ratio (%) |
|---|---|
| GM1 | 56.8 |
| GM2 | 69.0 |
| GM3 | 50.0 |
| GD1b | 43.4 |
| GD3 | 51.2 |
| Gb4 | 26.8 |
| asialo-GM1 | 72.5 |
| LacCer | 11.2 |
| GalCer | 14.4 |
| GlcCer | 7.3 |

### (4) Optimum pH value

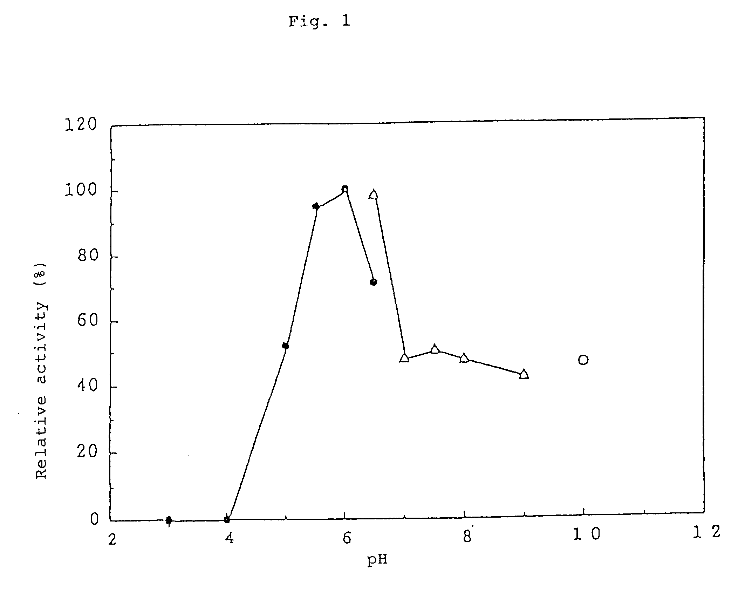

As Fig. 1 shows, the enzyme of the present invention exhibits a high activity at about pH 5 to 7. In the assay of the activity, a 50 mM acetate buffer solution, a 50 mM phosphate buffer solution and a 50 mM glycine buffer solution are used respectively at pH 3.0 to 6.5, pH 6.5 to 9.0 and pH 10. Fig. 1 shows the optimum pH value of the enzyme of the present invention wherein the ordinate refers to the relative activity (%) and the abscissa refers to pH. In Fig. 1, 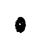 stands for the acetate buffer solution, △ stands for the phosphate buffer solution and 0 stands for the glycine buffer solution.

### (5) Optimum temperature

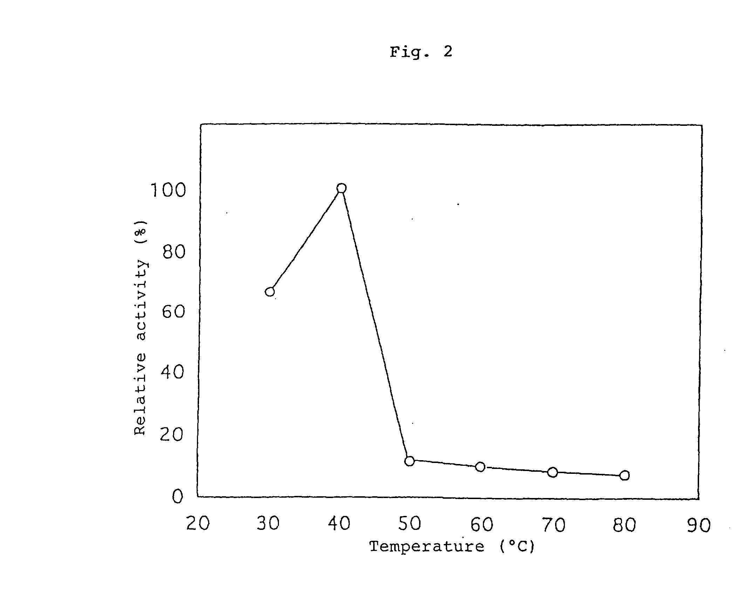

As Fig. 2 shows, the enzyme of the present invention exhibits the maximum activity at about 40 °C. That is to say, Fig. 2 shows the optimum temperature of the enzyme of the present invention wherein the ordinate refers to the relative activity (%) and the abscissa refers to the temperature (°C).

### (6) pH stability

The enzyme of the present invention is maintained at each definite pH value at 5 °C for 16 hours. After returning the pH value to 6.0, the enzyme activity is measured to thereby examine the pH stability of the enzyme. As the buffer solution, a 50 mM acetate buffer solution, a 50 mM phosphate buffer solution, a 50 mM tris-hydrochloride buffer solution and a 50 mM glycine buffer solution are used respectively at pH 3.5 to 6.0, pH 6.0 to 8.0, pH 8.0 to 9.0 and pH 9.0 to 9.5.

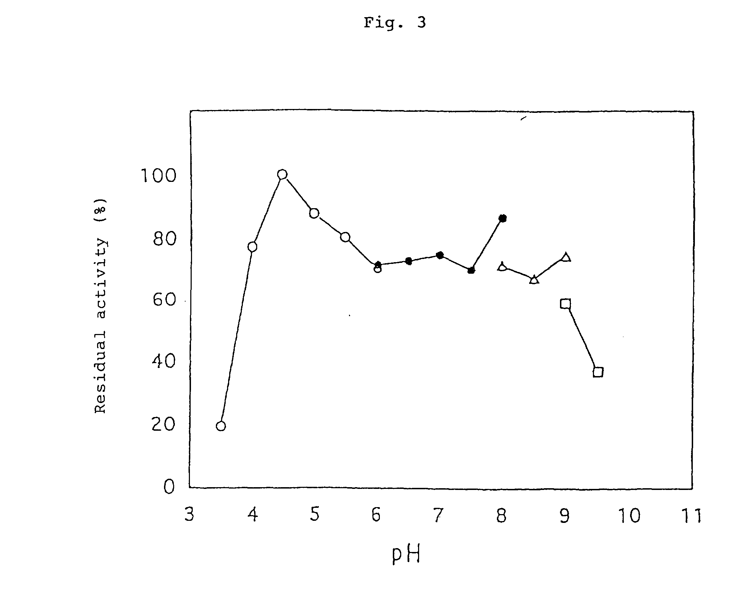

As Fig. 3 shows, the enzyme of the present invention remains stable within a range of pH 4 to 9. Namely, Fig. 3 shows the pH stability of the enzyme of the present invention wherein the ordinate refers to the residual activity (%) and the abscissa refers to the pH. In Fig. 3, 0 stands for the acetate buffer solution, 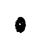 stands for the phosphate buffer solution, △ stands for the tris-hydrochloride buffer solution and □ stands for the glycine buffer solution.

### (7) Heat stability

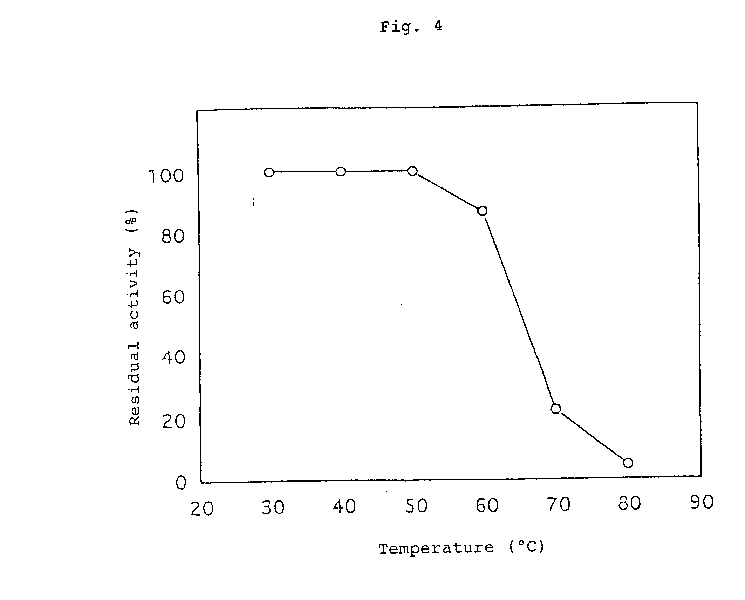

An examination on the heat stability of the enzyme of the present invention indicates that it sustains 90 % of the activity after treating at 60 °C for 30 minutes, as shown in Fig. 4. That is to say, Fig. 4 shows the heat stability of the enzyme of the present invention wherein the ordinate refers to the residual activity (%) and the abscissa refers to the temperature (°C).

### (8) Molecular weight

As the result of SDS-polyacrylamide gel electrophoresis (SDS-PAGE), it is found out that the molecular weight of the enzyme of the present invention is about 52,000.

### (9) Identification of the structure of enzyme reaction product

The digestion product obtained by the enzyme of the present invention is identified by digesting asialo-GM1 with the enzyme, purifying the digestion product by reversed phase high performance liquid chromatography (HPLC) and then analyzing the purified product by fast atom bombardment mass spectrum (FAB-MS). Namely, 200 µl of an enzyme solution and 10 µl of toluene are added to 1 ml of a 50 mM acetate buffer solution (pH 6.0) containing 3 mg/ml of asialo-GM1 (C 18 : 0, d 18 : 1, molecular weight: 1,254) and 0.6 % of Triton X-100® and the mixture is reacted at 37 °C for 3 days. After the completion of the reaction, chloroform/methanol (2:1 by volume) is added to the reaction mixture in a 5-fold amount thereof. After partition, the upper layer is recovered and evaporated to dryness. The resulting residue is dissolved in 500 µl of chloroform/methanol/water (3:48:47 by volume) to thereby give a sample for the reversed phase column chromatography. An ODS-80T column (column size: 4.6 x 75 mm, manufactured by Tosoh Corporation) is employed therein. The flow rate is set to 1 ml/min and fractions are collected in 1.5 ml portions. After adding the sample to the column, 10 ml of methanol/water (6:4 by volume) is passed therethrough. Next, gradient elution is effected until the concentration of methanol reaches 100 % for 60 minutes. Finally, 5 ml of chloroform/methanol/water (60:30:4.5 by volume) is passed through the column. Fractions of lysoasialo-GM1 are collected to thereby give a purified preparation which is then subjected to the FAB-MS analysis (matrix: triethanolamine).

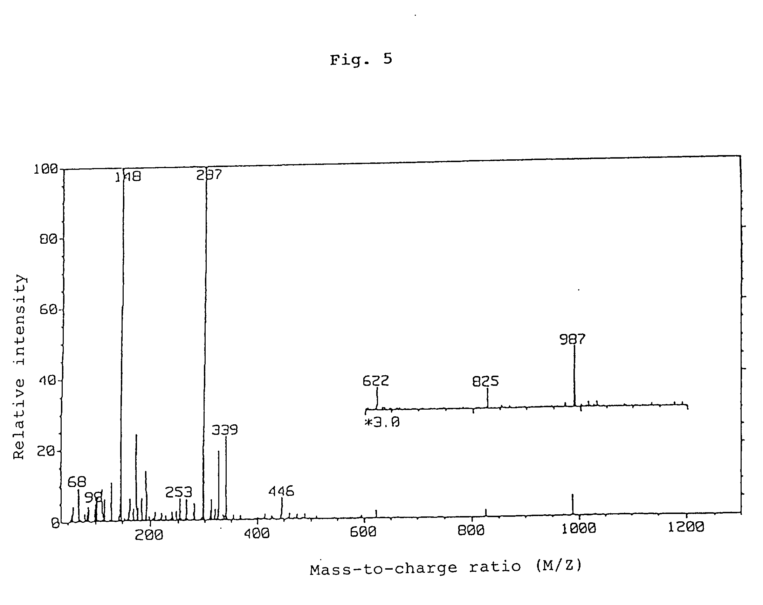

Fig. 5 shows the results. That is to say, Fig. 5 shows the FAB-MS data of the product obtained by digesting asialo-GM1 with the enzyme of the present invention. In Fig. 5, the ordinate refers to the relative intensity and the abscissa refers to the mass-to-charge ratio (M/Z). An magnification [600 - 1200 (M/Z)] is given at the center of Fig. 5. The signal position [(M-H)⁻] of asialo-GM1 (Gal-GalNAc-Gal-Glc-Cer, wherein Cer stands for ceramide; molecular weight: 1254) employed as the substrate is 1253, while that of lysoasialo-GM1 (Gal-GalNAc-Gal-Glc-Sph, wherein Sph stands for sphingosine base; molecular weight: 988) formed as the digestion product is 987.

As Fig. 5 shows, signal 987 corresponding to the molecular weight 988 of lysoasialo-GM1 is obtained as the strongest one. Also, Fig. 5 shows signal 825, which indicates that Gal has liberated from the nonreducing end of the carbohydrate chain moiety of lysoasialo-GM1, and another signal 622, which indicates that N-acetylgalactosamine (GalNAc) has further liberated therefrom. Based on these data, it is suggested that the product of the reaction by the enzyme of the present invention is a lysoglycolipid having the carbohydrate chain moiety of asialo-GM1 in its carbohydrate chain moiety.

In addition to the results of the FAB-MS analysis as described above, there have been proved the following facts.
(I) The product obtained by the digestion with the enzyme of the present invention is positive in the ninhydrin reaction.
(II) The carbohydrate chain moiety of asialo-GM1 is formed by treating this product with endoglycoceramidase (EC. 3.2.1.123) which is an enzyme capable of hydrolyzing a glycoside bond between a carbohydrate chain and a ceramide or a carbohydrate chain and a sphingosine.
(III) This carbohydrate chain moiety is negative in the ninhydrin reaction, which indicates that the acetyl group of GalNAc has not liberated therefrom.

Based on these results, it has been clarified that the enzyme of the present invention acts on a ceramide in the molecule of a glycolipid and hydrolyzes the ceramide moiety into a sphingosine base and a fatty acid, thus catalyzing a reaction for the formation of a lysoglycolipid and a fatty acid.

In the production of a lysosphingolipid using the enzyme of the present invention, any sphingolipid can be used as a substrate as long as it is digested by the enzyme of the present invention. Examples of the sphingolipids include acidic glycolipids such as sulfatide or ganglioside, for example, GQ1, GT1, GD1, GD3, GM1 or GM3, neutral glycolipids such as globoside, asialo-GM1 or cerebroside, and sphingophospholipids such as sphingomyelin.

Various lysosphingolipids can be obtained by suspending these substrate in a buffer and performing the enzymatic reaction using the enzyme of the present invention. The enzymatic reaction can be carried out, for example, under such conditions as the amount of the enzyme of about 40 mU/200 µl of the reaction mixture, a substrate concentration in the reaction mixture of from 1 to 20 mg/ml, a temperature of from 37 to 40 °C and a pH of from 5.0 to 6.0. An acetate buffer is preferably used and its concentration is adjusted to 25 mM. In addition, it is preferable to add a surfactant such as Triton X-100® in the reaction mixture to give a final concentration of 0.8 %. The reaction time is not particularly limited as long as a lysosphingolipid is produced from the substrate. The reaction is generally completed for about 30 minutes to 3 days. After the completion of the reaction, the reaction product can be separated from unreacted sphingolipid by ODS reverse phase column chromatography. Elution can be carried out using chloroform/methanol/water (5:4:1 by volume) as an eluent. Eluates can be monitored by HPTLC analysis. HPTLC can be carried out in a conventional manner using chloroform/methanol/10 % acetic acid (5:4:1 by volume) as a developing solvent. Color development can be carried out by the orcinol-sulfuric acid method for detection of a glycolipid and a lysoglycolipid, and by the Coomassie Brilliant Blue method for detection of sphingomyelin and lysosphingomyelin. For exclusive detection of a lysosphingolipid, the ninhydrin method can be used.

As described above, a lysosphingolipid can be produced using the enzyme of the present invention.

A lysosphingolipid derivative can be produced by introducing a suitable substituent to the thus-obtained lysosphingolipid by a substitution reaction.

For example, an acylated derivative can be obtained by esterifying a fatty acid and N-hydroxysuccinic acid imide in the presence of dicyclohexylcarbodiimide and reacting the resulting product with a lysosphingolipid to introduce a fatty acid thereto. Alternatively, a fatty acid chloride may be synthesized and reacted with a lysosphingolipid to introduce a fatty acid thereto.

Fluorescence-labeled sphingolipid derivatives (fluorescence-labeled neosphingolipids) can be synthesized by fluorescence-labeling of an amino group of a sphingosine moiety of the lysosphingolipid obtained using the enzyme of the present invention. Examples of fluorescent substances include dansyl chloride, 4-fluoro-7-nitrobenzofurazan, NBD-F and 10-pyrenedecanic acid.

As described above, the present invention provides a glycolipid ceramide deacylase having an extremely wide substrate specificity and a method of producing a lysosphingolipid using the glycolipid ceramide deacylase. Use of the enzyme of the present invention makes it possible to prepare lysosphingolipids from various sphingolipids. These lysosphingolipids thus obtained can be further converted into sphingolipid derivatives by utilizing the free amino acids thereof by, for example, reintroducing a labeled fatty acid thereinto, directly labeling the same with a fluorescent substance or binding to a carbohydrate chain-free protein (e.g., albumin) in a conventional manner. These derivatives are useful as a substrate for assaying a carbohydrate-relating enzyme, as a ligand in affinity chromatography for purification, as an antigen against an anti-sphingolipid antibody or in a study for revealing the function of sphingolipids. Further, the lysosphingolipid obtained using the enzyme of the present invention serves as a substrate or a reagent useful in the field of cell technology such as elucidation of intracellular metabolism and transport route of sphingolipids or function of sphingolipids in the cells.

To further illustrate the present invention in greater detail, the following example will be given. However this example is intended to illustrate an example of the embodiment of the invention and is not to be construed to limit the scope of the same.

### EXAMPLE 1

*Pseudomonas* sp. TK-4 (BP-5096) was inoculated to an inoculum size of 2 % into a liquid medium, which contained 0.5 % of peptone, 0.1 % of yeast extract, 0.2 % of NaCl and 0.1 % of asialo-GM1, and incubated therein at 30 °C for 48 hours. After the completion of the incubation, the culture medium was centrifuged at 6,000 rpm for 60 minutes. Thus the cells were eliminated and a culture supernatant was obtained. The subsequent procedures were all performed at 5 °C. Ammonium sulfate was added to the culture supernatant to achieve 80 % saturation. Then it was allowed to stand overnight and centrifuged at 6,000 rpm for 60 minutes. The precipitate thus obtained was collected and dissolved in a small amount of a 20 mM acetate buffer solution (pH 6.0) containing 0.2 % of Lubrol®. Next, it was dialyzed against the same buffer solution overnight. After the completion of the dialysis, the enzyme solution was subjected to gel filtration chromatography with the use of a Toyopearl® HW-55F column (column size: 40 x 300 mm; manufactured by Tosoh Corporation). Elution was carried out using as an eluent a 20 mM acetate buffer solution (pH 6.0) containing 0.2 % of Lubrol® and 0.2 M of NaCl at a flow rate of 1 ml/min to collect fractions in 5 ml portions. Active fractions were collected and subjected to column chromatography using a DEAM column (column size: 2.3 x 150 mm; manufactured by J. T. Baker) which had been equilibrated with a 20 mM acetate buffer solution (pH 6.0) containing 0.2 % of Lubrol®. Elution was carried out using the same buffer solution at a flow rate of 2 ml/min to collect unadsorbed fractions. The resulting fractions were applied to a CM-5PW column (column size: 5 x 50 mm, manufactured by Tosoh Corporation) at a flow rate of 0.5 ml/min to collect fractions in 1.5 ml portions. The development was performed by linear gradation elution of from 0 to 1 M NaCl to collect active fractions which was subjected to gel filtration chromatography using a TSK-G300SW column (manufactured by Tosoh Corporation). Elution was carried out using a 20 mM acetate buffer solution (pH 6.0) containing 0.2 % of Lubrol® and 0.2 M of NaCl at a flow rate of 1.5 ml/min to collect fractions in 1.5 ml portions. Active fractions were collected and subjected to column chromatography of hydroxyapatite (column size: 1.4 x 70 mm) which had been equilibrated with a 2 mM phosphate buffer solution (pH 7.0) containing 0.2 % of Lubrol®. Elution was performed by linear gradient elution of from the starting buffer solution to a 400 mM phosphate buffer solution (pH 7.0). The active fractions were collected to give a purified enzyme. It was confirmed that this purified enzyme hydrolyzed asialo-GM1 to form lysoasialo-GM1 and a fatty acid.

### EXAMPLE 2

### (1) Production of lysoasialo-GM1

Asialo-GM1 was used as a sphingolipid. It was prepared from bovine brain in accordance with the method described in Methods in Enzymology, 83, 139-191 (1982). The purified enzyme obtained in Example 1 (40 mU) was added to 200 µl of 25 mM acetate buffer (pH 6.0) containing 2.5 mg/ml of asialo-GM1 and 0.8 % Triton X-100® and the resulting mixture was incubated at 37 °C for 3 days to perform the reaction. After the completion of the reaction, partition was carried out by adding chloroform/methanol (2:1 by volume) in a 5-fold amount of the reaction mixture. The upper layer is recovered and evaporated to dryness. The resulting residue was dissolved in 500 µl of chloroform/methanol/water (3:48:47 by volume) and subjected to ODS reverse phase column chromatography to thereby separate the reaction product and unreacted sphingolipid (asialo-GM1). An ODS-80T column (4.6 x 75 mm, Tosoh Corporation) was used therefor. The flow rate was set to 1 ml/min and fractions were collected in 1.5 ml portions. Elution was carried out using chloroform/methanol/water (5:4:1 by volume). Eluates were monitored by HPTLC analysis. HPTLC was carried out using chloroform/methanol/10 % acetic acid (5:4:1 by volume) as a developing solvent making use of the orcinol-sulfuric acid method for color development. For exclusive detection of a lysosphingolipid, the ninhydrin method was used.

Fractions containing lysoasialo-GM1 were collected to serve as a purified product and subjected to FAB-MS analysis (matrix: triethanolamine). The results are shown in Fig. 5. The ordinate and abscissa stand for relative intensity and mass-to-charge ratio (M/Z), respectively. Signal 987 which corresponds to molecular weight 988 of lysoasialo-GM1 was observed as the strongest signal. Fig. 5 also shows signal 825 indicating that Gal was liberated from the nonreducing end of the carbohydrate chain of lysoasialo-GM1 and signal 622 indicating that N-acetylgalactosamine (GalNAc) was further liberated therefrom.

The purified product is positive in the ninhydrin reaction. When the purified product was treated with endoglycoceramidase, the carbohydrate chain moiety of asialo-GM1 was formed. The resulting carbohydrate chain moiety was negative in the ninhydrin reaction.

From these results, the purified reaction product was found to be lysoasialo-GM1.

### (2) Production of lysosphingomyelin

The same procedure as in Example 2 (1) was repeated using sphingomyelin (manufactured by Matreya) as a sphingolipid except for using silica gel 60 column in place of ODS-80T column under the same conditions as in the case of using ODS-80T column and performing color development by the Coomassie Brilliant Blue® method in place of the orcinol method.

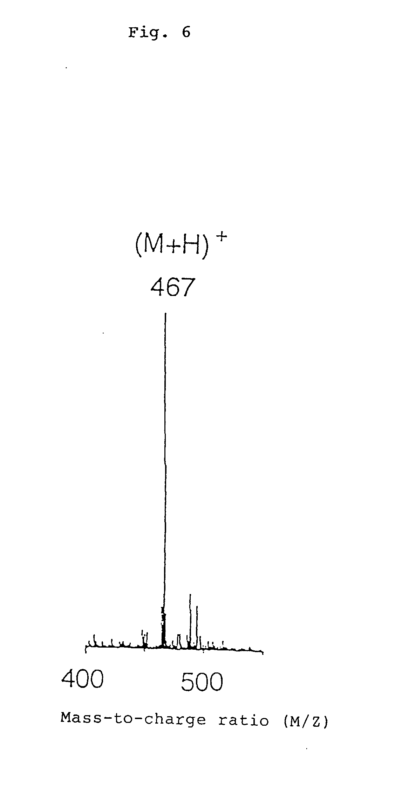

The results of FAB-MS analysis of the reaction product are shown in Fig. 6. The ordinate and abscissa stand for relative intensity and mass-to-charge ratio (M/Z), respectively. Fig. 6 is a magnification in the range from 400 to 550 (M/Z).

As shown in Fig. 6, the strongest signal was signal 467 [(M+H⁺)] corresponding to the molecular weight 466 of lysosphingomyelin.

### EXAMPLE 3

### Synthesis of lysosphingolipid derivatives

### (1) Introduction of single chain fatty acid to lysosphingolipid

A fatty acid was introduced to a lysosphingolipid by reacting synthesized fatty acid chloride with a lysosphingolipid (reacylation). Lyso-GM1 was prepared in the same manner as described in Example 2 (1) and used as a lysosphingolipid. As a fatty acid, molecular species of C2:0, C14:0, C16:0, C18:0, C22:0 and C24:0 were used. Two to three molar equivalents of a fatty acid to lyso-GM1 was put into a small egg-plant flask and 5 to 10 ml of thionyl chloride was added thereto. Attaching a condenser, the flask was heated at about 80 °C in water bath under reflux. In this instance, the reflux condenser was equipped with a glass tube packed with calcium chloride at its tip for blocking the outside air. After the completion of the reaction, thionyl chloride was removed under nitrogen gas stream in a draft and the flask was allowed to stand in a desiccator with potassium hydroxide for 1 to 2 hours. With confirming no smell of thionyl chloride, diethyl ether was added to the flask to dissolve the reaction product contained therein. Further, 1 µmol of lyso-GM1 dissolved in 1 ml of 0.3 M sodium hydrogencarbonate solution was added thereto followed by stirring for 2 to 3 hours. The reaction was monitored by TLC. After the completion of the reaction, the reaction mixture was dialyzed against ultrapure water to obtain reacylated GM1. According to this method, any fatty acid could be introduced to lyso-GM1 in a yield of about 90 % irrespective of the length of the carbon chain of the fatty acid.

### (2) Synthesis of fluorescence-labeled neosphingolipid

Fluorescence-labeled sphingolipid derivatives (fluorescence-labeled neosphingolipids) were synthesized by fluorescence-labeling an amino group of the sphingosine moiety of a lysosphingolipid. Lyso-GM1 was prepared in the same manner as described in Example 2 (1) and used as a lysosphingolipid.

For labeling with dansyl chloride, lyso-GM1 (1 µmol) was dissolved in 1 ml of 0.2 M sodium hydrogencarbonate solution and an equivalent volume of 0.25 % dansyl chloride in acetone was added thereto. The resulting mixture was allowed to stand in the dark at 37 °C for 1 hour and dialyzed against ultrapure water.

On the other hand, labeling with NBD-F was carried out in the following manner. Lyso-GM1 (0.5 nmol) was dissolved in 500 µl of 0.1 M borate buffer (pH 8.0), 500 µl of 20 mM NBD-F/ethanol solution was added thereto and the resulting mixture was incubated in water bath at 60 °C for 1 minute. Immediately thereafter, the reaction mixture was cooled in ice and dialyzed against ultrapure water at 4 °C for about 2 hours.

In both cases, it was necessary to carry out the reaction in the dark.

The thus synthesized fluorescence-labeled neosphingolipid was assayed by TLC using chloroform/methanol/10 % acetic acid (5:4:1 by volume) as a developing solvent. The yield of dansylated GM1 (Dansyl-II3 NeuAca-Gg4-sphingosine) was almost 100 %, while that of NBDGM1 (NBD-II3NeuAcα-Gg4-sphingosine) was 70-80 %.

## Claims

1. A glycolipid ceramide deacylase having the following physicochemical properties of:
(1) acting on a ceramide in the molecule of a sphingoglycolipid and hydrolyzing it into a sphingosine base and a fatty acid, thus forming a lysoglycolipid and a fatty acid;
(2) acting on neutral glycolipids and acidic glycolipids;
(3) having an optimum pH value range of from 5 to 7;
(4) having an optimum temperature of about 40 °C;
(5) being stable within a pH value range of from 4 to 9 when treated at 5 °C for 16 hours; and
(6) being stable when treated at 60 °C for 30 minutes.

2. The glycolipid ceramide deacylase according to claim 1, derivable from *Pseudomonas* sp. TK-4 (FERM BP-5096).

3. A method of producing a lysosphingolipid which comprises treating a sphingolipid with the glycolipid ceramide deacylase of claim 1 and recovering a lysosphingolipid from the reaction mixture.

4. A method of producing a glycolipid ceramide deacylase which comprises cultivating Pseudomonas sp. TK-4 (FERM BP-5096) in a medium and recovering the enzyme from the medium.

5. A method of producing a lysosphingolipid derivative which comprises treating a sphingolipid with the glycolipid ceramide deacylase of claim 1, recovering a lysosphingolipid from the reaction mixture and subjecting the lysosphingolipid to a substitution reaction.

6. The method of producing a lysosphingolipid derivative according to claim 5, wherein the substitution reaction is an acylation reaction.

7. The method of producing a lysosphinoglipid derivative according to claim 5, wherein the substitution reaction is carried out by fluoresence-labeling an amino group of a sphingosine moiety of the lysosphingolipid.

## Patentansprüche

1. Glykolipid-Ceramiddeacylase mit den folgenden physiko-chemischen Eigenschaften:
(1) Aktivität gegenüber einem Ceramid in einem Sphingoglykolipidmolekül und dessen Hydrolyse in eine Sphingosinbase und eine Fettsäure, wodurch ein Lysoglykolipid und eine Fettsäure gebildet werden;
(2) Aktivität gegenüber neutralen Glykolipiden und sauren Glykolipiden;
(3) Optimum-pH-Wert-Bereich von 5 bis 7;
(4) Optimum-Temperatur bei etwa 40°C;
(5) Stabilität innerhalb eines pH-Wert-Bereichs von 4 bis 9 bei Behandlung bei 5°C während 16 Stunden; und
(6) Stabilität bei Behandlung bei 60°C während 30 Minuten.

2. Glykolipid-Ceramiddeacylase nach Anspruch 1, erhältlich aus *Pseudomonas sp.* TK-4 (FERM BP-5096).

3. Verfahren zur Herstellung eines Lysosphingolipids, umfassend die Behandlung eines Sphingolipids mit der Glykolipid-Ceramiddeacylase nach Anspruch 1 und Isolierung eines Lysosphingolipids aus dem Reaktionsgemisch.

4. Verfahren zur Herstellung einer Glykolipid-Ceramiddeacylase, umfassend das Kultivieren von *Pseudomonas* sp. TK-4 (FERM BP-5096) in einem Medium und Isolierung des Enzyms aus dem Medium.

5. Verfahren zur Herstellung eines Lysosphingolipid-Derivats, umfassend die Behandlung eines Sphingolipids mit der Glykolipid-Ceramiddeacylase nach Anspruch 1, Isolierung eines Lysosphingolipids aus dem Reaktionsgemisch und Unterwerfen des Lysosphingolipids einer Substitutionsreaktion.

6. Verfahren zur Herstellung eines Lysosphingolipid-Derivats nach Anspruch 5, dadurch **gekennzeichnet**, daß die Substitutionsreaktion eine Acylierungsreaktion ist.

7. Verfahren zur Herstellung eines Lysosphingolipid-Derivats nach Anspruch 5, dadurch **gekennzeichnet**, daß die Substitutionsreaktion durch Fluoreszensmarkierung einer Aminogruppe einer Sphingosineinheit des Lysosphingolipids durchgeführt wird.

## Revendications

1. Céramide-désacylase pour glycolipides ayant les propriétés physico-chimiques suivantes :
(1) agir sur un céramide dans la molécule d'un sphingoglycolipide et l'hydrolyser en une base sphingosine et un acide gras, en formant ainsi un lysoglycolipide et un acide gras;
(2) agir sur les glycolipides neutres et les glycolipides acides ;
(3) avoir un domaine de pH optimal de 5 à 7;
(4) avoir une température optimale d'environ 40°C;
(5) être stable dans un domaine de pH de 4 à 9 lors de traitement à 5°C pendant 16 h ; et
(6) être stable lors de traitement à 60°C pendant 30 min.

2. Céramide-désacylase pour glycolipides selon la revendication 1, susceptible d'être dérivée de Pseudomonas sp. TK-4 (FERM BP-5096).

3. Procédé de production d'un lysosphingolipide, lequel comprend le traitement d'un sphingolipide par la céramide-désacylase pour glycolipides selon la revendication 1 et la récupération d'un lysosphingolipide dans le mélange réactionnel.

4. Procédé de production d'une céramide-désacylase pour glycolipides, lequel comprend la culture de Pseudomonas sp. TK-4 (FERM BP-5096) dans un milieu et la récupération de l'enzyme dans le milieu.

5. Procédé de production d'un dérivé de lysosphingolipide, lequel comprend le traitement d'un sphingolipide par la céramide-désacylase pour glycolipides selon la revendication 1, la récupération d'un lysosphingolipide dans le mélange réactionnel et la soumission du lysosphingolipide à une réaction de substitution.

6. Procédé de production d'un dérivé de lysosphingolipide selon la revendication 5, où la réaction de substitution est une réaction d'acylation.

7. Procédé de production d'un dérivé de lysosphingolipide selon la revendication 5, où la réaction de substitution est mise en oeuvre par marquage par fluorescence d'un groupe aminé d'une fraction sphingosine du lysosphingolipide.
